# EUROPEAN PATENT APPLICATION

(11) **EP 3 216 379 A1**
(43) Date of publication of application: **13.09.2017**
(21) Application number: 15855349.5
(22) Date of filing: 08.10.2015
(51) Int. Cl.: A61B 1/00, G02B 23/24

(54) **ENDOSCOPE**

(30) Priority: 31.10.2014 JP 2014223563
(71) Applicant: Olympus Corporation, Tokyo 192-8507 (JP)
(72) Inventor: HAMAZAKI, Masanori, Tokyo 192-8507 (JP)
(74) Representative: Schicker, Silvia
(86) International application number: PCT/JP2015/078621
(87) International publication number: WO 2016/067882

(57) **Abstract**

The endoscope includes an insertion section 27 to be inserted into a cavity to be observed, a distal end member provided at the distal end of the insertion section 27 and having a hole 48, a tubular member inserted in the hole 48, a fixing member 51 engageable with one member selected from between the distal end member and the tubular member and configured to fix the other member by clamping the other member with the one member in the extending direction of the hole.

## Description

### FIELD

The present invention relates to an endoscope that enables observation of the inside of a cavity.

### BACKGROUND

An endoscope is known wherein an objective lens unit is fixed to the distal end of an insertion section with screws. In the endoscope, an imaging unit is secured to a distal end main body by fixing screws to the circular lens frame of the imaging unit in a direction in which the circle of the lens frame is deformed.

### CITATION LIST

### PATENT LITERATURE

1. Jpn. Pat. Appln. KOKAI Publication No. 2005-161071

### SUMMARY

### TECHNICAL PROBLEM

Where screws are fixed to a lens frame in a lens frame-deforming direction, the screws should not be inserted too much. If the screws are inserted excessively, the lens frame may be deformed, and the adhesive between the lens frame and the lens may exfoliate. As a result, moisture may enter the lens frame, causing the lens to fog. On the other hand, if the screws are not sufficiently inserted, the lens frame tends to be shaky and may be deformed when a shock is applied to the inserted portion of the screws. For this reason, the conventional method for securing a lens unit has room for improvement. In this case, the lens inside the lens frame may crack. For this reason, the conventional method for securing a lens unit has room for improvement.

An object of the present invention is to provide an endoscope which enables a unit to be firmly fixed to a distal end main body with no need to fix screws in a radial direction of the unit.

### SOLUTION TO PROBLEM

To achieve the object, an endoscope comprises an insertion section to be inserted into a cavity to be observed, a distal end member provided at the distal end of the insertion section and having a hole, a tubular member inserted in the hole, a fixing member engageable with one member selected from between the distal end member and the tubular member and configured to fix the other member selected from between the distal end member and the tubular member by fixing it with the one member in an extending direction of the hole.

### ADVANTAGEOUS EFFECTS OF INVENTION

With the above structure, the present invention provides an endoscope which enables a unit to be firmly fixed to a distal end main body with no need to fix screws in a radial direction of the unit.

### BRIEF DESCRIPTION OF THE DRAWINGS

FIG. 1 is a perspective view showing the entire structure of an endoscope apparatus according to the first embodiment.
FIG. 2 is a front view showing how the distal-end hard portion of the endoscope apparatus looks like when viewed from the end face side.
FIG. 3 is a sectional view taken in a plane along the longitudinal direction of the distal-end hard portion depicted in FIG. 2.
FIG. 4 is a sectional view depicting the F4 portion indicated in FIG. 3 in an enlarged scale.
FIG. 5 is a sectional view taken along line F5-F5 shown in FIG. 3.
FIG. 6 is a sectional view taken along line F6-F6 shown in FIG. 3.
FIG. 7 is a sectional view taken along line F7-F7 shown in FIG. 3.
FIG. 8 is a sectional view taken in a plane along the longitudinal direction of the distal-end hard portion of an endoscope apparatus according to the second embodiment.
FIG. 9 is a sectional view taken along line F9-F9 shown in FIG. 8.
FIG. 10 is a sectional view taken along line F10-F10 shown in FIG. 8.
FIG. 11 is a sectional view taken in a plane along the longitudinal direction of the distal-end hard portion of an endoscope apparatus according to the third embodiment.
FIG. 12 is a sectional view taken along line F12-F12 shown in FIG. 11.
FIG. 13 is a sectional view taken along line F13-F13 shown in FIG. 11.
FIG. 14 is a perspective view showing the distal-end hard portion and the jig and tool of an endoscope apparatus according to the fourth embodiment.
FIG. 15 is a sectional view taken in a plane along the longitudinal direction of the distal-end hard portion of the endoscope apparatus depicted in FIG. 14.
FIG. 16 is a sectional view taken along line F16-F16 shown in FIG. 15.
FIG. 17 is a sectional view taken along line F17-F17 shown in FIG. 15.
FIG. 18 is a sectional view taken along line F18-F18 shown in FIG. 15.
FIG. 19 is a sectional view showing the distal-end hard portion and the jig and tool of an endoscope apparatus according to a modification of the third embodiment.

### DETAILED DESCRIPTION

### [First Embodiment]

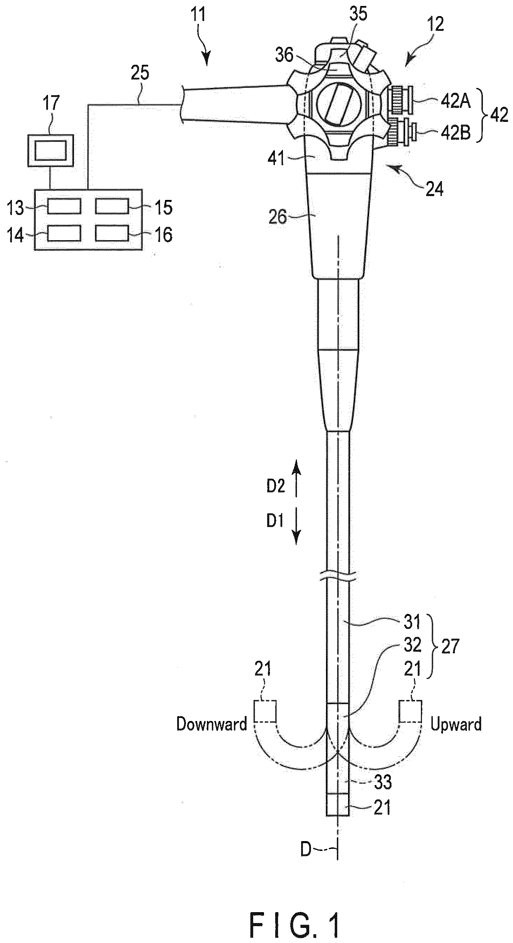

An endoscope apparatus according to the first embodiment will be described with reference to FIGS. 1 to 7. FIG. 1 shows the entire structure of an endoscope apparatus according to the present invention. As shown in FIG. 1, the endoscope apparatus 11 comprises an endoscope 12, a controller 13 which performs control including image processing, a light source 14, an air supply/water supply/suction apparatus 15, a keyboard 16 and a monitor 17.

Under the control of the controller 13, the light source 14 supplies light to an illumination lens 22, located at the distal-end hard portion 21 (mentioned later) of the endoscope 12. Under the control of the controller 13, the air supply/water supply/suction apparatus 15 supplies air or water to the nozzle 23 of the distal-end hard portion 21 of the endoscope 12, or sucks a liquid or tissues through a channel 45. The image processor of the controller 13 performs image processing for an image signal of an examinee photographed through an objective lens 43 of the distal-end hard portion 21 of the endoscope 12, and displays that image on the monitor 17.

As shown in FIG. 1, the endoscope 12 comprises an operation section 24, a universal code 25 for coupling the operation section 24 and the controller 13, a grip 26 adjacent to the operation section 24, and an insertion section 27 extending from the grip 26 and configured to be inserted into a cavity of an observation target (an examinee or an apparatus). The endoscope 12 is connected to the controller 13, light source 14, and air supply/water supply/suction apparatus 15 by means of the universal code 25.

In FIG. 1, arrow D1 indicates the distal direction of the longitudinal direction D of the insertion section 27, and arrow D2 indicates the proximal direction of the longitudinal direction. The insertion section 27 includes a flexible tube portion 31 which is elongated and flexible, a bending portion 32 located at the distal end of the flexible tube portion 31, and a distal-end hard portion 21 located at the distal end of the bending portion 32 and functioning as a distal end member.

The bending portion 32 contains a plurality of cylindrical bending pieces 33 arranged in the longitudinal direction D of the insertion section 27. A pin is clamped between the adjacent bending pieces 33 to enable the being pieces 33 to change their angles, and a plurality of bending pieces 33 form a joint enabling the bending portion 32 to bend. The outer circumference of the bending portion 32 is covered with an outer tube 34 (described later). A pair of first wires for permitting the bending portion 32 to bend in a U direction or in a D direction opposite thereto, and a pair of second wires for permitting the bending portion 32 to bend in an R direction or in an L direction opposite thereto, are inserted through the bending pieces 33. The first wires and the second wires are fixed to the bending piece 33 located at the most distal end of the insertion section 27. In the operation section 24, the paired first wires are fixed to the first pulley of a first dial unit 35. Likewise, in the operation section 24, the paired second wires are fixed to the second pulley of a second dial unit 36. The bending pieces 33, first wires and second wires enable the bending portion 32 to be moved in the UP direction, DOWN direction, RIGHT direction, LEFT direction, or any direction obtained by combining those directions.

As shown in FIG. 1, the operation section 24 comprises a case 41 made, for example, of a synthetic resin, a first dial unit 35 projected from the case 41 and rotatable relative to the case 41, a second dial unit 36 projected from the case 41 and rotatable relative to the case 41, and a button section 42 provided for the case 41. The button section 42 includes a first button 42B (an air supply/water supply button) operated when the air or water is supplied to the distal-end hard portion 21 of the endoscope through the nozzle 23, and a second button 42A (a suction button) operated when the distal-end hard portion of the endoscope performs suction through the channel 45.

The first dial unit 35 is operated when the bending portion 32 should be bent in the U direction (upward) or the D direction (downward). When the user operates the UD knob of the first dial unit 35, the first pulley is rotated, pulling one of the first wires and releasing the tension of the other first wire. As a result, the bending portion 32 is bent in the U direction or the D direction.

The second dial unit 36 is operated when the bending portion 32 should be bent in the R direction (rightward) or the L direction (leftward). When the user operates the RL knob of the second dial unit 36, the second pulley is rotated, pulling one of the second wires and releasing the tension of the other second wire. As a result, the bending portion 32 is bent in the R direction or the L direction.

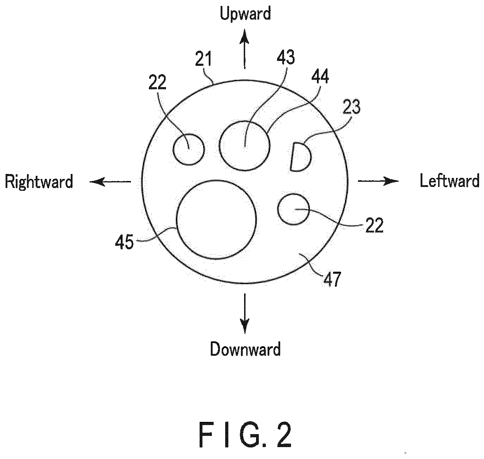

As shown in FIG. 2, the distal-end hard portion 21 comprises: a unit 44 including an objective lens 43 and serving as a cylindrical member; a channel 45 used for sucking a liquid and a tissue from a cavity and enabling a treatment tool to be inserted therethrough for treatment; an illumination lens 22; and a nozzle 23 for supplying cleaning the water to clean the distal end face of the distal-end hard portion 21.

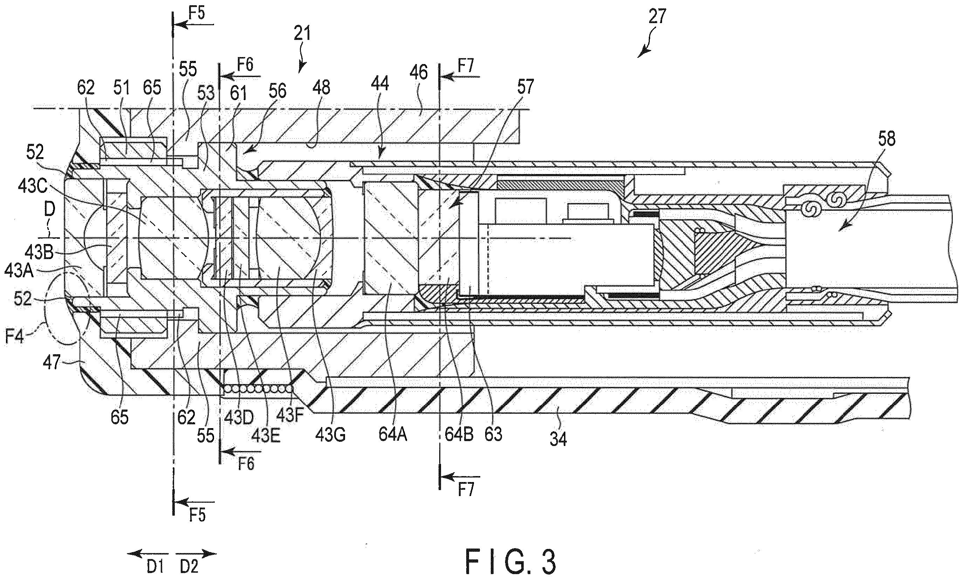

As shown in FIG. 3, the distal-end hard portion 21 comprises: a metallic and substantially cylindrical distal-end hard portion main body 46 (a distal end member); a distal end cover 47 for covering the distal end of the distal-end hard portion main body 46; an outer cover 34 made of rubber and configured to cover the outer circumference of the distal-end hard portion 21; a hole 48 formed in the distal-end hard portion main body 46; a unit 44 received in the hole 48; a fixing member 51 for fixing the unit 44 to the distal-end hard portion main body 46; and an adhesive 52 interposed between the lens frame 53 of the unit 44 and the distal end cover 47.

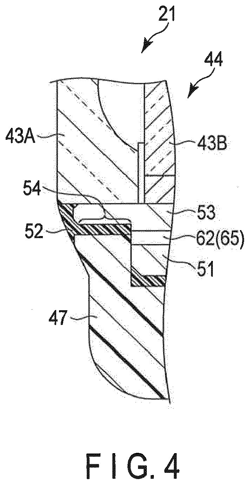

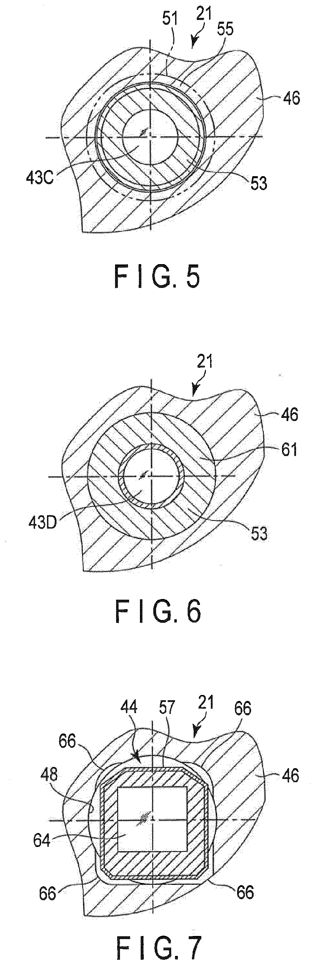

The distal end cover 47 is made, for example, of a synthetic resin material and shaped like a cap. As shown in FIG. 4, the adhesive 52 seals the gap 54 between the lens frame 53 and the distal end cover 47. As shown in FIG. 3, the hole 48 is open in the distal end face of the distal-end hard portion main body 46. The hole 48 extends in the longitudinal direction D of the insertion section 27. The hole 48 has a substantially circular cross section. The distal-end hard portion main body 46 includes a first projection 55. As shown in FIGS. 3 and 5, the first projection 55 is projected from the inner circumferential face of the hole 48 toward the unit 44 (i.e., toward the center of the hole 48). The first projection 55 is in the form of a ring and extends along the whole circumference of the unit 44. The first projection 55 may have substantially a ring shape which is discontinuous in part.

As shown in FIG. 3, the unit 44 includes a so-called objective lens section 56, an imaging unit 57 fixed to the objective lens section 56, and a cable which bundles a plurality of electric lines extending from the imaging unit 57 to the operation section 24.

The objective lens section 56 includes a substantially cylindrical lens frame 53, a plurality of objective lenses 43 assembled within the lens frame 53 (e.g., first to seventh objective lenses 43A to 43G), a second projection 61 projected outward from the outer peripheral portion of the lens frame 53, and a first screw portion 62 formed on the outer circumferential (peripheral) face of the lens frame 53 on the distal direction (D1) side. As shown in FIGS. 3 and 6, the second projection 61 is in the form of a ring and provided along the whole circumference of the outer peripheral portion (lens frame 53) of the unit 44. The second projection 61 can be brought into contact with the first projection 55. The second projection 61 may have substantially a ring shape which is discontinuous in part. The objective lens section 56 has a substantially cylindrical shape.

As shown in FIG. 3, the imaging unit 57 is provided such that a CCD 63 is opposed to the objective lens section 56. The imaging unit 57 has a substantially quadrangular prism shape. The imaging unit 57 includes the CCD 63, and cover glasses 64A and 64B covering the CCD 63. The imaging unit 57 is fixed to the objective lens section 56 by means of an adhesive, for example.

The fixing member 51 is made, for example, of a metallic material and shaped like a ring (nut). The fixing member 51 has a second screw portion 65 formed on the inner circumferential face thereof, and the second screw portion 65 is threadably engageable with the first screw portion 62. The fixing member 51 can be secured to the unit 44. In the state where the fixing member 51 is secured to the unit 44, the first projection 55 of the distal-end hard portion main body 46 can be clamped between the fixing member 51 and the second projection 61. The fixing member 51 is aligned with the first projection 55 and the second projection 61 in the longitudinal direction D of the insertion section 27.

A method for assembling the distal-end hard portion 21 of the insertion section 27 and the related members of the present embodiment will be described with reference to FIGS. 3 to 7. The unit 44 is inserted into the hole 48 of the distal-end hard portion main body 46 from the proximal direction (D2) side of the longitudinal direction D. By this operation, the first projection 55 of the distal-end hard portion main body 46 strikes against the second projection 61 of the unit 44. In this state, the lens frame 53 (first screw portion 62) of the unit 44 is projected from inside the first projection 55.

The fixing member 51 on which the second screw portion 65 is formed is fastened to the first screw portion 62 in the distal direction D1 of the longitudinal direction D. When the fixing member 51 is fastened, it is in contact with the first projection 55, and the first projection 55 of the distal-end hard portion main body 46 is clamped between the second projection 61 of the unit 44 and the fixing member 51. As a result, the unit 44 is firmly fixed to the distal-end hard portion main body 46. Lastly, the distal end cover 47 is laid over the distal-end hard portion main body 46, thereby completing the assembling operation. As shown in FIG. 7, the hole 48 of the distal-end hard portion main body 46 has escape sections 66 by which the distal-end hard portion main body 45 is located away from the corners of the unit 44. The escape sections 66 are formed at the positions where the imaging unit 57 of the unit 44 passes.

According to the first embodiment, the endoscope 12 comprises an insertion section 27 to be inserted into a cavity to be observed, a distal end member provided at the distal end of the insertion section 27 and having a hole 48, a tubular member inserted in the hole 48, a fixing member 51 engageable with one member selected from between the distal end member and the tubular member and configured to fix the other member selected from between the distal end member by clamping it with the one member in the extending direction of the hole.

The fixing member 51 is engaged with the tubular member, and the tubular member is fixed to the distal end member by clamping the distal end member between the tubular member and the fixing member 51. The distal end member includes a first projection 55 projected from the inner circumferential face of the hole 48, and the tubular member includes a second projection 61 projected from the outer circumferential face of the tubular member. The first projection 55 is clamped between the fixing member 51 and the second projection 61.

With this structure, the tubular member can be firmly secured by the fixing member 51. Since the first projection 55 of the tubular member is clamped between the fixing member 51 and the second projection 61 of the tubular member, the tubular member is not loose and can be firmly secured to the tubular member.

The fixing member 51 is aligned with the first projection 55 and the second projection 61 in the longitudinal direction D of the insertion section 27. With this structure, the fixing member 51, the first projection 55 and the second projection 61 are not arranged in a radial direction of the insertion section 27, so that the radii of the insertion section 27 and the distal-end hard portion main body 46 are small.

The second projection 61 extends along substantially the whole circumference of the outer peripheral portion. With this structure, a shock externally applied to the unit 44 (tubular member) is diffused by the second projection 61.

The fixing member 51 is in the form of a ring. This structure ensures that the unit 44 can be secured firmly. The unit 44 (tubular member) has a first screw portion 62 formed on the outer periphery thereof, and the fixing member 51 has a second screw portion 65 formed on the inner circumferential face thereof and being engageable with the first screw portion 62. With this structure, pressure can be uniformly applied to the second projection 61 and the unit 44 continuous with the second projection 61 when the first projection 55 is clamped between the fixing member 51 and the second projection 61. Even if a shock is exerted on the insertion section 27, stress does not concentrate on a particular portion of the unit 44. Since damage to the unit 44 is thus prevented, the reliability of the endoscope 12 can be enhanced.

The unit 44 (tubular member) includes a tubular lens frame 53 on which the second projection 61 is provided, and lenses incorporated inside the lens frame 53. With this structure, the lens frame 53 is not deformed when the unit 44 is fixed. Where the unit 44 includes fragile elements such as lenses, damage to such fragile elements can be prevented to a maximal degree, and yet the unit 44 can be firmly secured.

The unit 44 (tubular member) includes an imaging unit 57 facing a lens. With this structure, even where the unit 44 includes an electronic component such as the imaging unit 57, damage to such fragile electronic component can be prevented to a maximal degree, and yet the unit 44 can be firmly secured to the distal-end hard portion main body 46.

### [Second Embodiment]

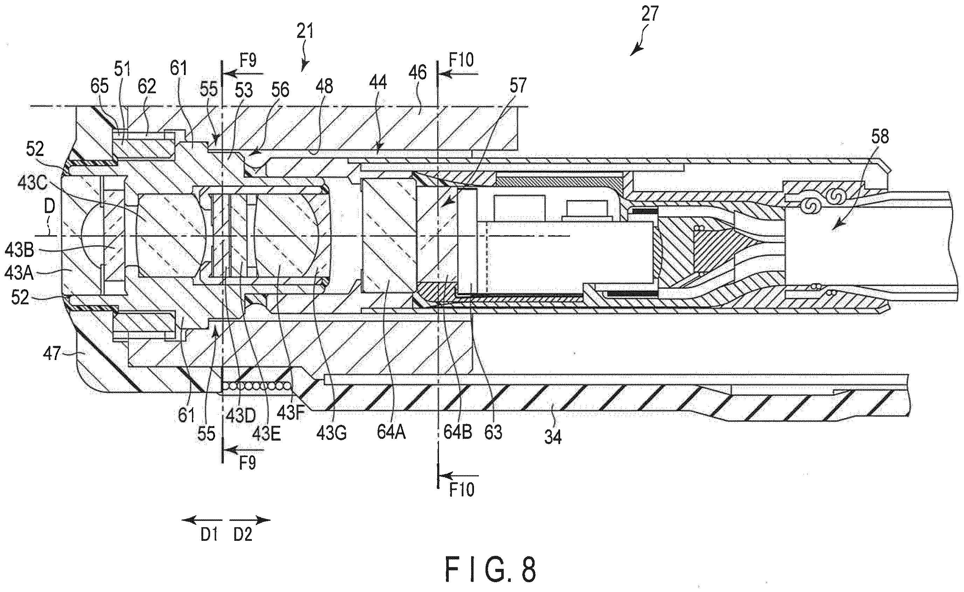

An endoscope apparatus according to the second embodiment will be described with reference to FIGS. 8 to 10. The endoscope apparatus of the second embodiment differs from that of the first embodiment in that the second projection 61 of the unit 44 is clamped between the first projection 55 of the distal-end hard portion main body 46 and the fixing member 51. The other features are similar to those of the first embodiment. A description will therefore be given mainly of how the second embodiment differs from the first embodiment. As for the features common to the first embodiment, a description and illustration of them will be omitted.

As shown in FIG. 8, a distal-end hard portion 21 comprises a metallic and substantially cylindrical distal-end hard portion main body 46, a distal end cover 47 for covering the distal end of the distal-end hard portion main body 46, an outer cover 34 for covering the outer circumference of the distal-end hard portion 21, a hole 48 formed in the distal-end hard portion main body 46, a unit 44 received in the hole 48, a fixing member 51 for fixing the unit 44 to the distal-end hard portion main body 46, and an adhesive 52 interposed between the lens frame 53 of the unit 44 and the distal end cover 47.

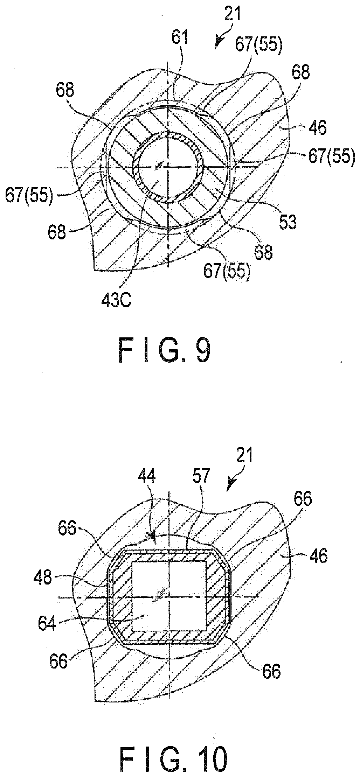

The distal-end hard portion main body 46 (distal end member) includes a first projection 55, and a first screw portion 62 formed on the inner circumferential face of the hole 48 at a position which is on the distal end side of the first projection 55. The first projection 55 is projected from the inner circumferential face of the hole 48 toward the unit 44 (toward the center of the hole 48). As shown in FIG. 9, unlike the first projection 55 of the first embodiment, the first projection 55 of the second embodiment is provided discretely. That is, the first projection 55 includes a plurality of extended portions 67 (e.g., four extended portions 67) protruding toward the center of the hole 48. The distal-end hard portion main body 46 is provided with recesses 68 at positions between the adjacent extended portions 67. The recesses 68 are provided at four respective positions in such a manner that the recesses 68 correspond to the four corners of the imaging unit 57 of the unit 44. As will be described below, when the unit 44 is inserted into the hole 48 from the distal direction (D1) side, the imaging unit 57 passes the hole 48 and the recesses 68 and comes to a position which is on the distal direction (D2) side of the first projection 55. As shown in FIG. 8, the first screw portion 62 is formed on the inner circumferential face of the hole 48 at a position which is most distal in the distal direction D1 and which corresponds to the objective lens section 56 of the unit 44.

The unit 44 (tubular member) includes a so-called objective lens section 56, an imaging unit 57 fixed to the objective lens section 56, and a cable 58 which bundles a plurality of electric lines extending from the imaging unit 57 to the operation section 24.

The objective lens section 56 includes a substantially cylindrical lens frame 53, a plurality of objective lenses 43 assembled within the lens frame 53 (e.g., first to seventh objective lenses 43), and a second projection 61 projected outward from the outer peripheral portion of the lens frame 53. The second projection 61 is in the form of a ring (a flange) and corresponds to the whole circumference of the outer peripheral portion (lens frame 53) of the unit 44. The second projection 61 can be brought into contact with the first projection 55. The second projection 61 may have substantially a ring shape which is discontinuous in part. The objective lens section 56 has a substantially cylindrical shape. The second embodiment differs from the first embodiment in that the lens frame 53 does not have a screw portion.

The fixing member 51 is made, for example, of a metallic material and shaped like a ring (bolt). The fixing member 51 has a second screw portion 65 formed on the inner circumferential face thereof, and the second screw portion 65 is threadably engageable with the first screw portion 62. The fixing member 51 can be secured to the distal-end hard portion main body 46. In the state where the fixing member 51 is secured to the distal-end hard portion main body 46, the second projection 61 of the unit 44 can be clamped between the fixing member 51 and the first projection 55 of the distal-end hard portion main body 46. The fixing member 51 is aligned with the first projection 55 and the second projection 61 in the longitudinal direction D of the insertion section 27.

A method for assembling the distal-end hard portion 21 of the insertion section 27 and the related members of the present embodiment will be described with reference to FIGS. 8 to 10. According to the present embodiment, the unit 44 is inserted into the hole 48 of the distal-end hard portion main body 46 from the distal direction (D1) side of the longitudinal direction D. Prior to the insertion, the cable 58 is made to pass through the hole 48 and the internal region of the insertion section 27 (the flexible tube portion 31 and the bending portion 32) continuous therewith, by means of a wire. By pulling the cable 58 from the proximal direction (D2) side of the insertion section 27, the unit 44 is pulled into the hole 48 of the distal-end hard portion main body 46 from the distal direction (D1) side. As a result, the second projection 61 of the unit 44 strikes against the first projection 55 of the distal-end hard portion main body 46. In this state, the first screw portion 62 of the distal-end hard portion main body 46 is exposed to the atmosphere.

The fixing member 51 on which the second screw portion 65 is formed is fastened to the first screw portion 62 in the distal direction D1 of the longitudinal direction D. When the fixing member 51 is fastened, it is in contact with the second projection 61, and the second projection 61 of the unit 44 is clamped between the first projection 55 of the distal-end hard portion main body 46 and the fixing member 51. As a result, the unit 44 is firmly fixed to the distal-end hard portion main body 46. As shown in FIG. 10, the hole 48 of the distal-end hard portion main body 46 has escape sections 66 by which the distal-end hard portion main body 45 is located away from the corners of the unit 44. The escape sections 66 are formed at the positions where the imaging unit 57 of the unit 44 passes.

According to the present embodiment, the fixing member 51 is engaged with the distal end member, and the tubular member is fixed to the distal end member by clamping the tubular member between the distal end member and the fixing member 51. The distal end member includes a first projection 55 projected from the inner circumferential face of the hole 48, and the tubular member includes a second projection 61 projected from the outer circumferential face of the tubular member. The second projection 61 is clamped between the fixing member 51 and the first projection 55.

With this structure, the second projection 61 of the tubular member is clamped between the fixing member 51 and the first projection 55 of the distal end member. As a result, the tubular member is not loose and can be firmly secured to the distal end member. With the above structure, the tubular member can be attached or detached from the hole 48 of the distal end member from the distal direction (D1) side. If the tubular member fails to function properly, it can be detached from the distal direction (D1) side and can be easily replaced with a new one. Accordingly, the endoscope 12 of the present embodiment is easy to maintain.

The fixing member 51 is aligned with the first projection 55 and the second projection 61 in the longitudinal direction D of the insertion section 27. With this structure, the fixing member 51, the first projection 55 and the second projection 61 are not arranged in a radial direction of the insertion section 27, so that the radii of the insertion section 27 and the distal-end hard portion main body 46 are small.

The second projection 61 extends along the whole circumference of the outer peripheral portion. With this structure, a shock externally applied to the unit 44 (tubular member) is diffused by the second projection 61.

The fixing member 51 is in the form of a ring. This structure ensures that the unit 44 (tubular member) can be secured firmly. The distal end member has a first screw portion 62 formed on the inner circumference face of the hole 48, and the fixing member 51 has a second screw portion 65 formed on the outer circumferential face thereof and being engageable with the first screw portion 62. With this structure, pressure can be uniformly applied to the second projection 61 and the unit 44 continuous with the second projection 61, when the second projection 61 is clamped between the fixing member 51 and the first projection 55. Even if a shock is exerted on the insertion section 27, stress does not concentrate on a particular portion of the unit 44. Since damage to the unit 44 is prevented, the reliability of the endoscope 12 can be enhanced.

### [Third Embodiment]

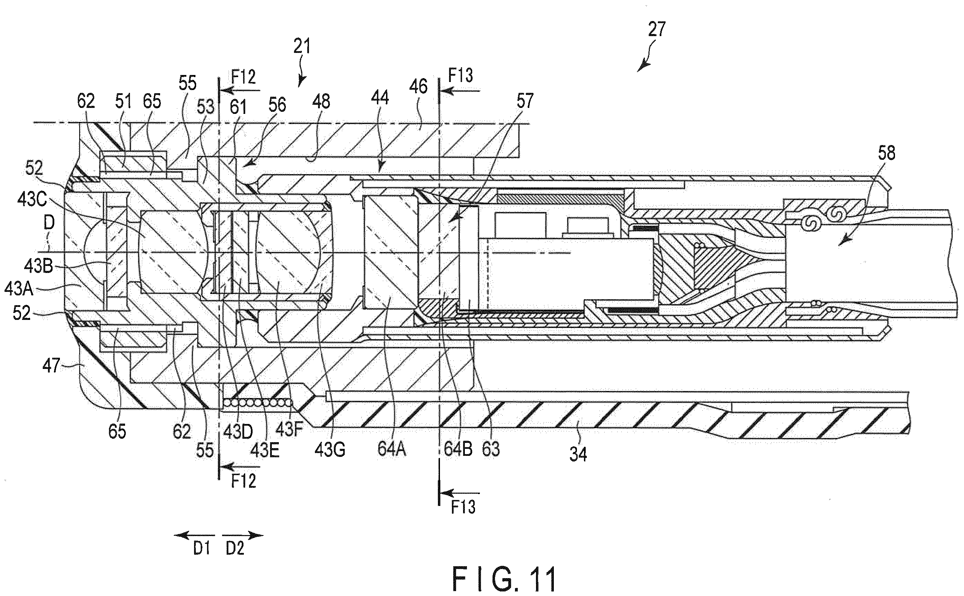

An endoscope apparatus according to the third embodiment will be described with reference to FIGS. 11 to 13. The endoscope apparatus 11 of the third embodiment differs from that of the first embodiment in that a rotation regulator 71 is provided between a distal-end hard portion main body 46 (distal end member) and a unit 44 (tubular member), but the other features are similar to those of the first embodiment. A description will therefore be given mainly of how the third embodiment differs from the first embodiment. As for the features common to the first embodiment, a description and illustration of them will be omitted.

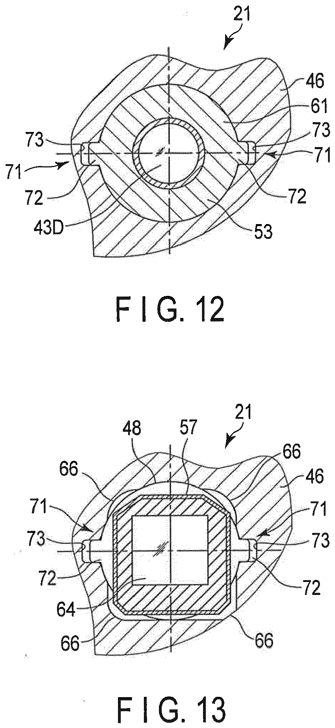

As shown in FIG. 11, a distal-end hard portion 21 comprises a metallic and substantially cylindrical distal-end hard portion main body 46, a distal end cover 47 for covering the distal end of the distal-end hard portion main body 46, an outer cover 34 for covering the outer circumference of the distal-end hard portion 21, a hole 48 formed in the distal-end hard portion main body 46, a unit 44 received in the hole 48, a fixing member 51 for fixing the unit 44 to the distal-end hard portion main body 46, a rotation regulator 71 (FIG. 12) provided between the distal-end hard portion main body 46 and the unit 44, and an adhesive 52 interposed between the lens frame 53 of the unit 44 and the distal end cover 47.

As shown in FIG. 12, the rotation regulator 71 is made by a convex portion 72 (a key portion) provided on the lens frame 53 of the unit 44, and a concave portion 73 (a key groove portion) formed in the distal-end hard portion main body 46 and depressed from the hole 48. Since the convex portion 72 fits in the concave portion 73, the rotation regulator 71 prevents the unit 44 from rotating relative to the distal-end hard portion main body 46.

A method for assembling the distal-end hard portion 21 of the insertion section 27 and the related members of the present embodiment will be described with reference to FIGS. 11 to 13. The unit 44 is inserted into the hole 48 of the distal-end hard portion main body 46 from the proximal direction (D2) side of the longitudinal direction D. At the time, the convex portion 72 is made to fit in the inside of the concave portion 73. When the second projection 61 of the unit 44 is brought into contact with the first projection 55 of the distal-end hard portion main body 46, the lens frame 53 (first screw portion 62) of the unit 44 is projected from inside the first projection 55.

The fixing member 51 on which the second screw portion 65 is formed is fastened to the first screw portion 62 from the distal direction (D1) side of the longitudinal direction D. When the fixing member 51 is secured, the rotation regulator 71 prevents the unit 44 from rotating together with the fixing member 51.

When the fixing member 51 is fastened, the first projection 55 of the distal-end hard portion main body 46 is clamped between the second projection 61 of the unit 44 and the fixing member 51. As a result, the unit 44 is firmly fixed to the distal-end hard portion main body 46. As shown in FIG. 13, the hole 48 of the distal-end hard portion main body 46 has escape sections 66 by which the distal-end hard portion main body 46 is located away from the corners of the unit 44. The escape sections 66 are formed at the positions where the imaging unit 57 of the unit 44 passes.

According to the present embodiment, the endoscope 12 is provided with the rotation regulator 71. The rotation regulator 71 is provided between the distal-end hard portion main body 46 and the unit 44 and prevents the unit 44 from rotating relative to the distal-end hard portion main body 46. With this structure, when the first screw portion 62 is fastened to the second screw portion 65 by rotating the fixing member 51, the unit 44 is prevented from rotating together with the fixing member 51. As a result, the fixing member 51 can be fastened easily.

According to the present embodiment, the rotation regulator 71 is provided between the distal-end hard portion main body 46 and the lens frame 53 of the unit 44, so that the assembling operation can be performed without applying a load to the other portions of the unit 44 (e.g., the portions around the imaging unit 57). As a result, damage to the unit 44 is prevented in the assembling operation.

### [Fourth Embodiment]

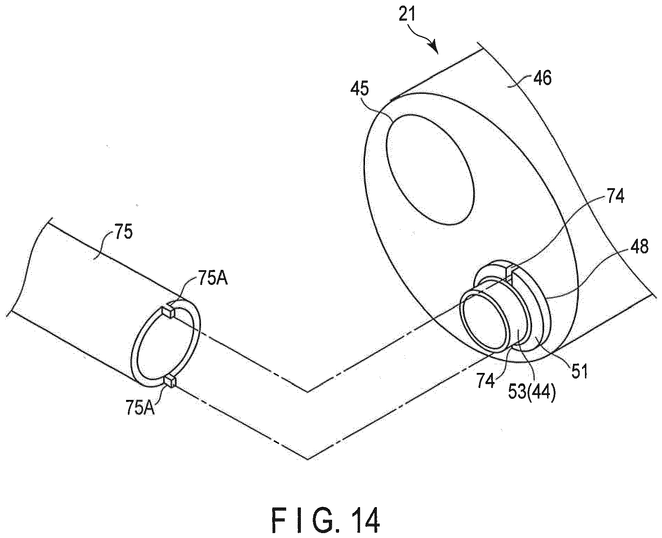

An endoscope apparatus according to the fourth embodiment will be described with reference to FIGS. 14 to 18. The endoscope apparatus 11 of the fourth embodiment differs from that of the second embodiment in that groove 74s for assisting the rotation of the fixing member 51 are provided and in that a rotation regulator 71 is provided between a distal-end hard portion main body 46 and a unit 44, but the other features are similar to those of the second embodiment. A description will therefore be given mainly of how the fourth embodiment differs from the second embodiment. As for the features common to the second embodiment, a description and illustration of them will be omitted.

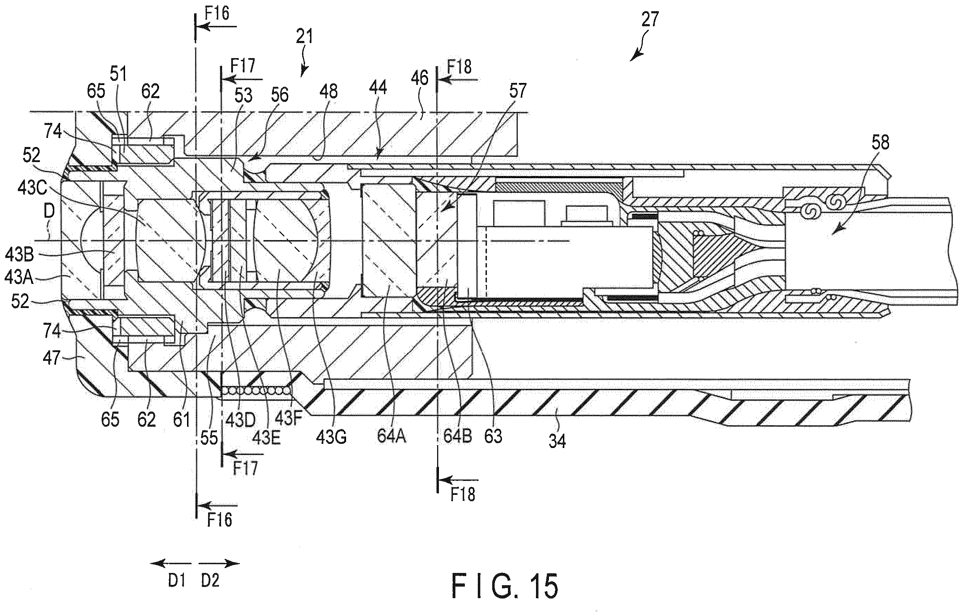

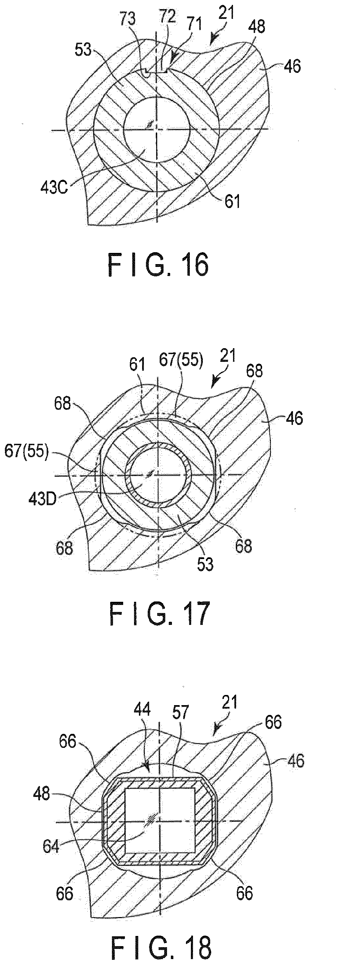

As shown in FIG. 15, a distal-end hard portion 21 comprises a metallic and substantially cylindrical distal-end hard portion main body 46, a distal end cover 47 for covering the distal end of the distal-end hard portion main body 46, an outer cover 34 for covering the outer circumference of the distal-end hard portion 21, a hole 48 formed in the distal-end hard portion main body 46, a unit 44 (tubular member) received in the hole 48, a fixing member 51 for fixing the unit 44 to the distal-end hard portion main body 46, a rotation regulator 71 (FIG. 16) provided between the distal-end hard portion main body 46 and the unit 44, and an adhesive 52 interposed between the lens frame 53 of the unit 44 and the distal end cover 47.

The fixing member 51 is made, for example, of a metallic material and shaped like a ring (bolt). The fixing member 51 has a second screw portion 65 formed on the outer circumferential face thereof and being engageable with the first screw portion 62. As shown in FIG. 14, the fixing member 51 has a pair of grooves 74 extending in a radial direction of the unit 44. Projected pieces 75A of a jig and tool 75 are insertable into the grooves 74. The paired grooves 74 are an example of engagement portions. As shown in FIGS. 15 and 17, the second projection 61 of the unit 44 can be clamped between the fixing member 51 and the first projection 55 of the distal-end hard portion main body 46. The jig and tool 75 is an example of a tool with which the fixing member 51 is rotated.

As shown in FIG. 16, the rotation regulator 71 is made by a concave portion 73 formed in the lens frame 53 of the unit 44, and a convex portion 72 projected from the distal-end hard portion main body 46 toward the hole 48. Since the convex portion 72 fits in the concave portion 73, the rotation regulator 71 prevents the unit 44 from rotating relative to the distal-end hard portion main body 46.

A method for assembling the distal-end hard portion 21 of the insertion section and the related members of the present embodiment will be described with reference to FIGS. 14 to 18. The unit 44 is inserted into the hole 48 of the distal-end hard portion main body 46 from the distal direction (D1) side of the longitudinal direction D. At the time, the convex portion 72 is made to fit in the inside of the concave portion 73. When the second projection 61 of the unit 44 is brought into contact with the first projection 55 of the distal-end hard portion main body 46, the lens frame 53 (first screw portion 62) of the unit 44 is projected from inside the first projection 55.

The fixing member 51 on which the second screw portion 65 is formed is fastened to the first screw portion 62 from the distal direction (D1) side of the longitudinal direction D. The projected pieces 75A of the jig and tool 75 are inserted into the grooves 74 of the fixing member 51. The fixing member 51 can be secured to the distal-end hard portion main body 46 by rotating the jig and tool 75. The jig and tool 75 enables the fixing member 51 to be fastened easily. When the fixing member 51 is fastened, the rotation regulator 71 prevents the unit 44 from rotating together with the fixing member 51.

When the fixing member 51 is secured, the second projection 61 of the unit 44 is clamped between the fixing member 51 and the first projection 55 of the distal-end hard portion main body 46. As a result, the unit 44 is firmly fixed to the distal-end hard portion main body 46. As shown in FIG. 18, the hole 48 of the distal-end hard portion main body 46 has escape sections 66 by which the distal-end hard portion main body 45 is located away from the corners of the unit 44. The escape sections 66 are formed at the positions where the imaging unit 57 of the unit 44 passes.

According to the present embodiment, the fixing member 51 includes an engagement portion, and a tool for rotating the fixing member 51 is engageable with the engagement portion. With this structure, the fixing member 51 can be fastened easily, using the tool. Accordingly, the endoscope 12 can be assembled efficiently.

In the present embodiment, the grooves 74 are formed in the fixing member 51. Instead of this structure, the fixing member 51 may have holes, and the fixing member 51 may be fastened, using a jig and tool 75 that is like a pair of compasses, as in a modification of the third embodiment mentioned below.

### (Modifications)

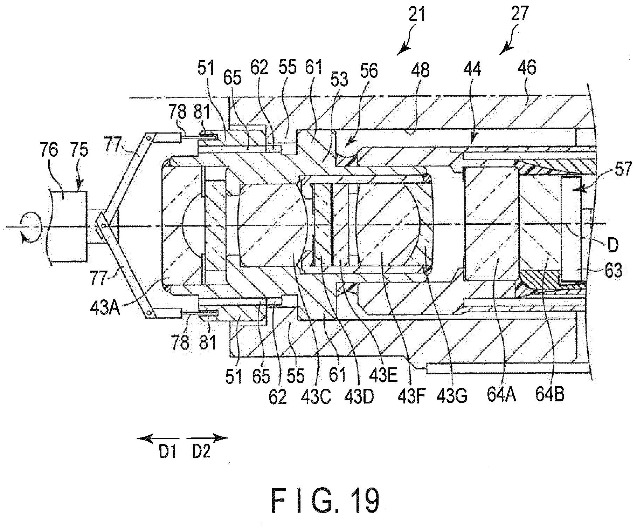

An endoscope apparatus according to a modification of the third embodiment will be described with reference to FIG. 19. A description will be given of how the modification differs from the third embodiment. As for the features common to the third embodiment, a description and illustration of them will be omitted.

According to the present modification, a jig and tool 75 can be used for fastening the fixing member 51, as in the fourth embodiment. According to the present modification, the jig and tool 75 comprises a rotating shaft 76, a pair of arms 77 which are like a pair of compasses, and pins 78 provided at the distal ends of the arms 77. The jig and tool 75 is an example of a tool with which the fixing member 51 is rotated.

The fixing member 51 is made, for example, of a metallic material and shaped like a ring (nut). The fixing member 51 has a second screw portion 65 formed on the inner circumferential face thereof and being engageable with the first screw portion 62. A pair of holes 81 are formed in the end face of the fixing member 51 by means of a drill or the like. The pins 78 of the jig and tool 75 can be inserted into the holes 81. The first projection 55 of the distal-end hard portion main body 46 (distal end member) can be clamped between the fixing member 51 and the second projection 61 of the unit 44 (tubular member). The holes 81 are an example of engagement portions.

A method for assembling the distal-end hard portion 21 of the insertion section 27 and the related members of the present embodiment will be described with reference to FIG. 19. The unit 44 is inserted into the hole 48 of the distal-end hard portion main body 46 from the proximal direction (D2) side of the longitudinal direction D. At the time, the convex portion 72 is made to fit in the inside of the concave portion 73. When the second projection 61 of the unit 44 is brought into contact with the first projection 55 of the distal-end hard portion main body 46, the lens frame 53 (first screw portion 62) of the unit 44 is projected from inside the first projection 55.

The fixing member 51 on which the second screw portion 65 is formed is fastened to the first screw portion 62 from the distal direction (D1) side of the longitudinal direction D. The pins 78 of the jig and tool 75 are inserted into the holes 81 of the fixing member 51. The fixing member 51 can be fastened to the distal-end hard portion main body 46 by rotating the jig and tool 75. The jig and tool 75 enables the fixing member 51 to be fastened easily. When the fixing member 51 is fastened, the rotation regulator 71 prevents the unit 44 from rotating together with the fixing member 51.

When the fixing member 51 is fastened, the first projection 55 of the distal-end hard portion main body 46 is clamped between the second projection 61 of the unit 44 and the fixing member 51. As a result, the unit 44 is firmly fixed to the distal-end hard portion main body 46.

According to the present modification, the fixing member 51 includes an engagement portion, and a tool for rotating the fixing member 51 is engageable with the engagement portion. With this structure, the fixing member 51 can be fastened easily, using the tool. Accordingly, the endoscope 12 can be assembled efficiently.

In the present modification, the holes 81 are formed in the fixing member 51. Instead of this structure, the fixing member 51 may have grooves 74, and the fixing member 51 may be fastened, using a jig and tool 75 which is cylindrical and which has a projected piece 78 at the distal end, as in the fourth embodiment described above.

The present invention is not limited to the above-described embodiments, and can be appropriately modified in practice, without departing from the gist of the invention. That is, in each of the above embodiments, the unit 44 was described as including an objective lens section 56 and an imaging unit 57. However, the unit 44 received in the hole 48 is not limited to this structure. The unit 44 received in the hole 48 may be an illumination lens unit including an illumination lens 22 and a light source. In addition, the method for securing the fixing member 51 to the unit 44 or distal-end hard portion main body 46 is not limited to a method using screws. The method for securing the fixing member 51 may be friction fit, forcible insertion or adhesion using an adhesive. In addition, one endoscope may be configured by properly combining the endoscopes of the above embodiments.

## Claims

1. An endoscope comprising:
an insertion section to be inserted into a cavity to be observed;
a distal end member provided at a distal end of the insertion section and having a hole;
a tubular member received in the hole; and
a fixing member engageable with one member selected from between the distal end member and the tubular member and configured to fix another member selected from between the distal end member and the tubular member by clamping said another member with the one member in an extending direction of the hole.

2. The endoscope according to claim 1, wherein the fixing member is engaged with the tubular member, and the tubular member is fixed to the distal end member by clamping the distal end member between the tubular member and the fixing member.

3. The endoscope according to claim 2, wherein the distal end member comprises a first projection projected from an inner circumferential face of the hole,
the tubular member comprises a second projection projected from an outer circumferential face of the tubular member, and
the first projection is clamped between the fixing member and the second projection.

4. The endoscope according to claim 3, wherein
the second projection is formed substantially along a whole circumference of the outer circumferential face of the tubular member.

5. The endoscope according to claim 4, wherein the fixing member is formed as a ring.

6. The endoscope according to claim 5, wherein the tubular member comprises a first screw portion formed on the outer circumferential face, and
the fixing member comprises a second screw portion formed on an inner circumferential face and being engageable with the first screw portion.

7. The endoscope according to claim 1, wherein the fixing member is engaged with the distal end member, and fixes the tubular member to the distal end member by clamping the tubular member between the distal end member and the fixing member.

8. The endoscope according to claim 7, wherein the distal end member comprises a first projection projected from an inner circumferential face of the hole,
the tubular member comprises a second projection projected from an outer circumferential face of the tubular member, and
the second projection is clamped between the fixing member and the first projection.

9. The endoscope according to claim 8, wherein the second projection is formed substantially along a whole circumference of the outer circumferential face of the tubular member.

10. The endoscope according to claim 9, wherein the fixing member is formed as a ring.

11. The endoscope according to claim 10, wherein the distal end member comprises a first screw portion formed on the inner circumferential face of the hole, and
the fixing member comprises a second screw portion formed on an outer circumferential face and being engageable with the first screw portion.

12. The endoscope according to claim 6, further comprising:
a rotation regulator provided between the distal end member and the tubular member and configured to prevent the tubular member from rotating relative to the distal end member.

13. The endoscope according to claim 12, wherein the fixing member includes an engagement portion with which a tool configured to rotate the fixing member is engageable.

14. The endoscope according to claim 13 wherein the tubular member comprises:
a lens frame on which the second projection is provided; and
a lens received within the lens frame.
